Europäisches Patentamt

European Patent Office

Office européen des brevets

(11) Publication number: **0 105 929**
**B1**

# EUROPEAN PATENT SPECIFICATION

(45) Date of publication of patent specification: **19.04.89**

(21) Application number: **83901977.5**

(22) Date of filing: **26.04.83**

(86) International application number:
**PCT/US83/00602**

(87) International publication number:
**WO 83/03753 10.11.83 Gazette 83/26**

(51) Int. Cl.⁴: **A 61 F 2/16**

(54) **POSTERIOR CHAMBER LENS IMPLANT.**

(30) Priority: **26.04.82 US 371541**

(43) Date of publication of application:
**25.04.84 Bulletin 84/17**

(45) Publication of the grant of the patent:
**19.04.89 Bulletin 89/16**

(84) Designated Contracting States:
**FR**

(56) References cited:
**WO-A-83/02224**
**US-A-4 092 473**
**US-A-4 198 714**
**US-A-4 242 760**
**US-A-4 244 060**
**US-A-4 253 199**
**US-A-4 304 012**

(73) Proprietor: **MYERS, William, D.**
**5855 Wingcroft Court**
**Birmingham, MI 48010 (US)**

(72) Inventor: **MYERS, William, D.**
**5855 Wingcroft Court**
**Birmingham, MI 48010 (US)**

(74) Representative: **Martin, Jean-Jacques et al**
**Cabinet REGIMBEAU 26, Avenue Kléber**
**F-75116 Paris (FR)**

Courier Press, Leamington Spa, England.

EP 0 105 929 B1

## Description

### Background of the Invention
### I - Field of the Invention

The present invention relates to a posterior chamber lens implant for use after extracapsular surgery.

### II - Description of the Prior Art

In extracapsular surgery, a surgical opening is formed through the front membrane or anterior capsule of the lens of the human eye and the cataracts and fluid within the interior of the lens is surgically removed. During such surgery, however, it is important to leave the posterior capsule of the lens intact so that it forms a barrier between the vitreous humor and the aqueous humor. Removal of the posterior capsule is known to result in a high incidence of retinal detachment as well as cystoid macular edema.

Following the removal of the cataracts, it is necessary to replace the human lens with an artificial lens implant in order to restore sight to the eye. Although these lens implants may be nested in the anterior chamber, i.e., behind the cornea, or in the pupil, it has been found that posterior chamber lens implants are medically superior to anterior chamber and pupil lens implants for a plurality of reasons.

The previously known posterior chamber lens implants comprise a substantially circular rigid optic having a continuous convex front surface, a rear surface and a substantially circular and continuous outer rear edge, means for securing said optic to the eye within the posterior chamber so that said outer rear edge abuts against the posterior capsule and so that the rear surface of said optic is spaced from the posterior capsule by a distance, according to the preamble of the unique claim.

The optic is typically secured in place in the posterior chamber by loops or haptics extending outwardly from the optic and sandwiched between the posterior capsule and anterior leads. A posterior chamber lens implant of this type is illustrated in US-A-4 244 060.

In a high incidence of cases after a period of time following implantation of the lens the posterior capsule becomes clouded and obscures the vision of the eye. In order to restore the vision to the eye after this has occurred, it is necessary to perform a posterior capsulotomy to remove the portion of the posterior capsule that is aligned with the optic.

In previously known methods to perform a posterior capsulotomy known as discission, a needle is inserted into the eye and used to punch a hole through the posterior capsule and behind the optic. In many cases, however, the posterior capsule becomes tough following the extracapsular surgery so that it is necessary to enter the eye with scissors in order to cut a hole in the posterior capsule. Both discission and membrane cutting, however, involve a significant risk of introducing bacteria or other contaminants into the eye which may ultimately result in loss of the eye.

Many attempts have been made to improve such a discission method, and US-A-4 244 060 gives an illustrative example of such attempts.

The above document discloses a posterior chamber lens implant which is plano-convex, which means that the lens body has a convex front surface and a planar rear surface. An annular lip or ridge is further provided, extending rearwardly from the lens body, a section of said ridge being missing to permit the insertion of a discission instrument therethrough: the purpose of the lip is to provide a barrier for preventing vitreous from coming forward into the anterior chamber after a discission has been done, and the space defined rearwardly is provided solely to facilitate a manual discission.

Known lens implants of the above type have a construction which implies several disadvantages:

the ridge provides a non-optical portion around the outer periphery of the lens body, so that the light passing therethrough is scattered, which interfers with patients vision;

in order to obtain an effective diameter optical body, i.e. the portion of the lens optic within the interior of the ridge, it is necessary to make the optic larger and to have a larger incision in the eye.

In the surgical procedure laser posterior capsulotomy, a laser is focused on the posterior capsule through the pupil. Upon activation of the laser, the laser burns an opening through the posterior capsule behind the optic thus restoring vision to the eye. The use of the laser in contrast with the previously known discission and membrane cutting is highly advantageous in that laser surgery is noninvasive and thus eliminates the possibility of introducing bacteria or other contaminants into the eye.

At present, however, laser posterior capsulotomy can be safely performed only on an anterior chamber or pupil lens implant. In a posterior chamber lens implant, the rear surface of the implant abuts substantially flatly against the posterior capsule, so that destruction of the posterior capsule by the laser may also result in destruction of the lens implant.

In particular, it will be understood that a lens implant as disclosed in US-A-4 244 060 is less suitable for a laser posterior capsulotomy than a lens implant according to the invention, since the posterior capsule may bulge outwardly towards the rear surface of the lens, so that the minimum spacing occurs between the posterior capsule and the center of the lens body.

### Summary of the Present Invention

The object of the present invention is to provide a posterior chamber lens implant which enables a subsequent laser posterior capsulotomy to be performed, if necessary, and which avoids the above disadvantages.

This is achieved by a posterior chamber lens implant of the above type, characterized in that said rear surface is a continuous concavely curved surface extending up to said outer rear edge and in that said distance is sufficient to safely allow a subsequent laser posterior capsulotomy, according to the characterizing portion of the unique claim.

With the lens positioned within the posterior chamber, only the outer periphery of the rear side of the optic abuts against the posterior capsule, and the remainder of the rear surface of the optic is spaced forwardly of the posterior capsule.

Furthermore, the above disadvantages do not exist with such a lens implant:

even if the lens decenters, there is no scattering of light since the entire optic provides a true optical body which properly refracts light (no ridge extending rearwardly);

it is only necessary that the outside diameter of the lens be equal to the desired optic diameter, since light is properly refracted by the entire optic;

due to the particular shape of the rear surface, the maximum spacing between the posterior surface of the optic and the posterior capsule following implantation occurs at the center of the lens, which is suitable for a laser posterior capsulotomy.

Brief Description of the Drawing

A better understanding of the present invention will be had upon reference to the following detailed description, when read in conjunction with the accompanying drawing, wherein like reference characters refer to like parts throughout the several views, and in which:

FIG. 1 is a fragmentary sectional view illustrating a known lens implant of the plano-convex type within the posterior chamber;

FIG. 2 is a fragmentary sectional view taken substantially along line 2-2 in FIG. 1, but relating to a lens implant having a continuous concavely curved rear surface, according to the present invention;

FIG. 3 is a view taken substantially along line 3-3 in FIG. 1, but relating to the lens implant of FIG. 2, and with parts removed for clarity;

FIG. 4 is a sectional view taken substantially along line 4-4 in FIG. 3, illustrating the concavely curved rear surface.

Detailed Description of a Preferred Embodiment of the Present Invention

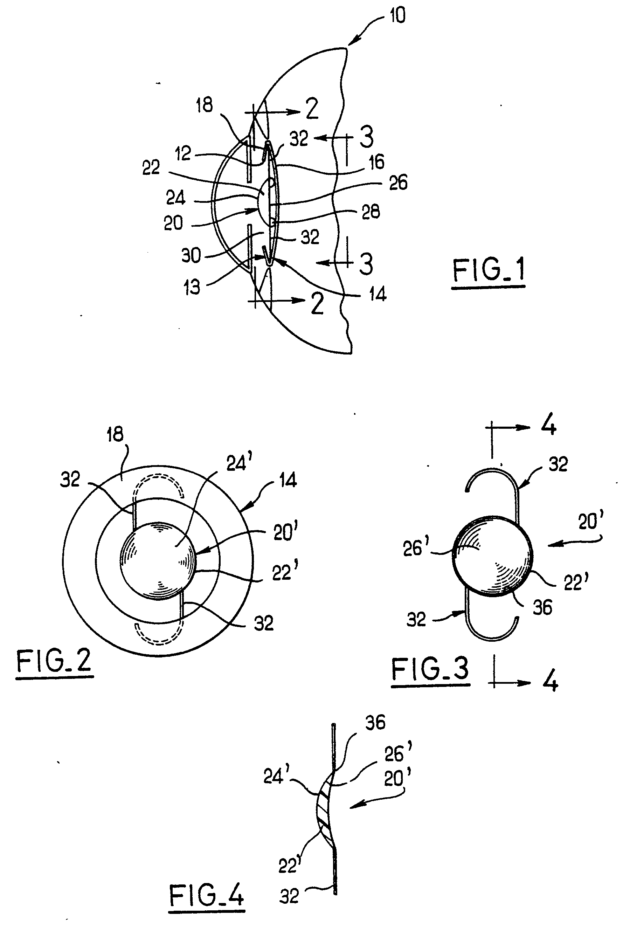

With reference first to FIG. 1, a human eye is there shown following extracapsular surgery. During extracapsular surgery, a circular opening 12 is formed in the anterior capsule 13 of the cataract capsule 14, or lens, and the cataracts are removed from the interior of the capsule 14. In doing so, the posterior capsule 16 of the lens 14 is left intact as well as an annular portion around the outer periphery of the anterior capsule 13 thus forming an annular anterior lead 18.

The known posterior chamber lens implant 20 illustrated in FIG. 1 comprises a central optic 22 having a front convex surface 24, and a planar rear surface 26. The optic 22, which is typically constructed of plastic, is generally designed to reproduce or approximate the optical qualities of the lens of the human eye. An annular ridge 28 is formed around the outer periphery on the rear surface 26 of the optic 22, so that the ridge 28 protrudes rearwardly from the optic 22 by a distance. This ridge 28 is substantially continuous and integrally constructed with the optic 22, as explained in US-A-4 244 060 illustrating a similar lens implant of the plano-convex type. The lens implant 20 is positioned within the posterior chamber 30 of the eye 10 so that the ridge 28 flatly abuts against the posterior capsule 16. Means are further provided to secure the lens implant 22 within the posterior chamber 30, such as one or more haptics 32 which are secured to and extend radially outwardly from the optic 22. An end portion of each haptic is positioned between the posterior capsule 16 and the anterior lead 18. Following extracapsular surgery, the anterior lead 18 folds against the posterior capsule 16 thus sandwiching the haptic end portions therebetween and securing the lens implant 20 in place. The use of haptics 32 positioned in between the posterior capsule 16 and anterior lead 18 is well known in the art.

With reference now to FIGS. 2 to 4, a preferred embodiment of the lens implant 20' of the present invention is there shown and comprises an optic 22' which is convexo-concave in shape. As such, the optic 22' includes a front convex surface 24' and a continuous concavely curved rear surface 26' extending between the outer rear edge of the lens implant 20'. Consequently, with the lens implant 20' positioned within the posterior chamber 30, only the outer periphery 36 of the optic 22' abuts against the posterior capsule 16 and spaces the central portion of the optic rear surface 26' forwardly from the posterior capsule 16.

The lens implant 20' can be secured within the posterior chamber by any conventional means, such as haptics 32 already mentioned with reference to the known lens implant illustrated in FIG. 1.

By spacing the rear surface 26' of the optic 32' forwardly from the posterior capsule 16 in such a manner, the present invention enables. the safe use of a laser to perform a posterior capsulotomy in the event that the posterior capsule subsequently becomes clouded or obscured. In practice, only a relatively small spacing, for example, one millimeter, is necessary between the rear surface of the lens implant and the posterior capsule in order to enable a laser posterior capsulotomy to be safely performed. As previously described, unlike discission and membrane cutting, laser posterior capsulotomy completely eliminates the possibility of bacterial infection or the introduction of other contaminants into the eye.

**Claim**

A posterior chamber lens implant (20') for a human eye for use after extracapsular surgery in which a posterior capsule is left substantially intact, said lens implant comprising a substantially circular rigid optic (22') having a continuous convex front surface (24'), a rear surface (26') and a substantially circular and continuous outer rear edge (36), means (32) for securing said optic to the eye within the posterior chamber so that said outer rear edge (36) abuts against the posterior capsule and so that the rear surface (26') of said optic is spaced from the posterior capsule by a distance, characterized in that said rear surface (26') is a continuous concavely curved surface extending up to said outer rear edge and in that said distance is sufficient to safely allow a subsequent laser posterior capsulotomy.

**Patentansprüch**

Linsenimplantat (20') für die hintere Kammer eines menschlichen Auges zur Verwendung nach extrakapsulärer Chirurgie, bei der eine hintere Kapsel im wesentlichen intakt gelassen wird, wobei das genannte Linsenimplantat eine im wesentlichen kreisförmige starre Optik (22') umfaßt, mit einer kontinuierlichen konvexen vorderen Oberfläche (24'), einer hinteren Oberfläche (26') und einem im wesentlichen runden und kontinuierlichen hinteren Außenrand (36), Einrichtungen (32) zum Befestigen der genannten Optik am Auge innerhalb der hinteren Kammer, so daß der genannte hintere Außenrand (36) gegen die hintere Kapsel angrenzt, und so, daß die hintere Oberfläche (26') der genannten Optik von der hinteren Kapsel mit Abstand angeordnet ist, dadurch gekennzeichnet, daß die genannte hintere Oberfläche (26') eine kontinuierliche konkav gekrümmte Oberfläche ist, die sich bis zu dem genannten hinteren Außenrand erstreckt, und dadurch, daß der genannte Abstand ausreichend ist, einen nachfolgenden Laserhinterkapselschnitt zu erlauben.

**Revendication**

Implant de lentille en chambre postérieure (20') pour l'œil humain, aux fins d'utilisation après chirurgie extra-capsulaire dans laquelle une capsule postérieure est laissée essentiellement intacte, ledit implant de lentille comprenant une optique rigide essentiellement circulaire (22') ayant une surface antérieure convexe et continue (24'), une surface postérieure (26') et un bord postérieur externe essentiellement circulaire et continu (36), des moyens (32) pour fixer ladite optique à l'œil sans la chambre postérieure de manière que ledit bord postérieur externe (36) bute contre la capsule postérieure et que la surface postérieure (26') de ladite optique soit espacée se la capsule postérieure d'une certaine distance, caractérisé en ce que ladite surface postérieure (26') est une surface courbée de façon concave et continue s'étendant jusqu' audit bord postérieur externe et en ce que ladite distance est suffisante pour permettre ultérieurement et en toute sécurité une capsulotomie postérieure au laser.

FIG_1

FIG_2

FIG_3

FIG_4